# EUROPEAN PATENT SPECIFICATION

(11) **EP 0 554 414 B1**
(45) Date of publication and mention of the grant of the patent: **07.01.1998**
(21) Application number: 92908548.8
(22) Date of filing: 26.03.1992
(51) Int. Cl.: A61K 39/23, C12N 15/86

(54) **METHOD FOR PRODUCING A SUBUNIT VACCINE AGAINST THE CANINE PARVOVIRUS AND OTHER RELATED VIRUSES**
VERFAHREN ZUR HERSTELLUNG VON UNTEREINHEITSIMPFSTOFF GEGEN HUNDEPARVOVIRUS UND VERWANDTEN VIREN
PROCEDE DE PRODUCTION D'UN VACCIN SOUS-UNITAIRE CONTRE LE PARVOVIRUS CANIN ET AUTRES VIRUS APPARENTES

(30) Priority: 26.03.1991 ES 9100844
(43) Date of publication of application: 11.08.1993
(73) Proprietor: INMUNOLOGIA Y GENETICA APLICADA, S.A., E-28037 Madrid (ES)
(72) Inventor: CORTES VALDES, Elena, E-28005 Madrid (ES); VELA OLMO, Carmen, E-28030 Madrid (ES); CASAL ALVAREZ, José, Ignacio, E-28039 Madrid (ES)
(86) International application number: PCT/ES92/00031
(87) International publication number: WO 92/17205

(56) References cited:
- EP-A- 0 341 611
- EP-A- 0 647 655
- WO-A-88/02026
- WO-A-90/05538
- US-A- 4 193 990
- JOURNAL OF GENERAL VIROLOGY, vol. 71, no. 11, November 1990, Society for General Microbiology (GB); J.C. MARTYN et al., pp. 2747-2753
- JOURNAL OF GENERAL VIROLOGY, vol. 73, no. 2, February 1992, Society for General Microbiology (GB); J.T. SALIKI et al., pp. 369-374
- Am. J. Vet. Res. 43, 1982, 2183 - 2187
- Cornell Vet. 72, 1982, 16 - 35
- Am. J. Vet. Res. 44, 1983, 169 - 175
- Vaccine 13, 1995, 1033 - 1037

## Description

### FIELD OF THE INVENTION

The present invention relates in general to viral proteins and to assays and vaccines using the same and, in particular, to a protein related to the major antigen (VP2) of the Canine Parvovirus (CPV) capsid. Such protein was produced in an expression vector of baculoviruses multiplied in a cell culture of a permissive host. The protein obtained with the instant invention is singularly characterized in forming empty chimeric capsids that are useful in vaccine formulation.

### BACKGROUND OF THE INVENTION

The Canine Parvovirus (CPV) belongs to the autonomous parvoviruses, causing severe enteritis in dogs of all ages and myocarditis in puppies less than 12 weeks old. CPV was first isolated in 1978 (Burtonboy, G. et al., Arch. Virol. 61:1-11, 1979; Appel et al., Vet. Rec. 105. 156-179, 1979). It is believed to have arisen as a natural variant of the feline panleukopenia virus (FPLV) or the mink enteritis virus (MEV). CPV infection is controlled using conventional vaccines based on live or inactivated viruses. However, since dogs are vaccinated before pregnancy maternal antibodies can block attenuated live vaccine replication. Autonomous parvoviruses provide a good vector for recombinant subunit vaccine production for several reasons, inter alia:
1. The capsid proteins are structurally simple (no glycosylation, phosphorylation or acetylation is required).
2. Humoral response appears to adequately control viral dissemination given the relative efficiency of inactivated vaccines.

Protein and DNA sequence studies and serologic studies show an enhanced antigenic and genetic homology between CPV, FPLV, MEV and the Raccoon Parvovirus (Tratschin et al., J. Gen Virol. 61:33-41. 1982. Carlsson et al., J. Virol., 55, 574-582, 1985. Parrish et al., Arch. Virol. 72, 267-278, 1982. Reed et al., J. Virol. 62:266-276, 1988). Despite this homology they are exquisitely specific in the "in vivo" host, although "in vitro" all viruses replicate in cat kidney cells (Appel et al., Vet. Rec. 105, 156-179, 1979. Trastschin et al., J. Gen. Virol., 61:33-41, 1982). The CPV capsid contains two proteins with broadly overlapping amino acid sequences, VP1 (82-84 KDa) and VP2 (67.70 KDa) (Paradiso et al. J. Gen. Virol. 39, 800-807, 1982. Surleraux et al. Arch Virol., 82, 233-240, 1984. J. Gen. Virol. 62, 113-125, 1982. Surleraux et al., Arch. Virol. 82, 233-240, 1984). The parvovirus capsid has a diameter of 22 nm and holds some 10 VP1 copies and some 60 VP2 copies (Wobble et al., Biochemistry 23, 6565-6569, 1984), arranged as either homo- or heterodimers (Paradiso, J. Virol., 46, 94-102, 1983) though the precise capsid structure is unknown. VP2 in full capsids (holding DNA) is preferentially broken down by proteolytic digestion into 63-67 KDa VP3 (Paradiso et al., Gen. Virol. 39, 800-807, 1982. Surleraux et al. Arch. Virol., 82, 233.240, 1984) after capsid assembly (Paradiso, J. Virol., 39, 800-807, 1981).

Our laboratory has of late been researching into the immunogenicity of various fragments of the proteins making up the CPV viral capsid which has resulted in new VP2 protein and VP2 and VP1 fragment based recombinant vaccines being described. These findings are summarised in Spanish patent number ES 2029938 for: "PRODUCTION OF CANINE PARVOVIRUS VACCINES BY VIRAL POLYPEPTIDE EXPRESSION IN E.coli OR BY CHEMICAL SYNTHESIS" with J.I. Casal et al. as inventors.

The said patent application relates to the expression of such products in E.coli bacterial vectors.

New large-scale protein production vectors have however been recently discovered based upon the replication of recombinant baculoviruses derived from the Autographa californica nuclear polyhedrosis virus (AcMNPV) in culture insect cells. The state of the art for these vectors is summed up in two scientific papers as follows:
1. LucKow, V.A. & Summers, M.D. (1988). Trends in the development of baculovirus expression vectors. Bio/Technology 6, 47-55.
2. J. Vialard et al. (1990). Synthesis of the membrane fusion and Hemagglutinin Proteins of Measles virus, using a novel baculovirus vector containing the β-galactosidase gene. J. Virol. 64. 37-50.

Furthermore, European Patent Application number 0 341 611 by Cornell Research Foundation, Inc. and Boyce Thompson Institute for Plant Research, Inc., describes the obtention of CPV subunit vaccines using a baculovirus expression vector other than that used in the present invention, and the said European Patent moreover fails to mention that the proteins obtained are capable of forming empty chimeric capsids thereby drawing a fundamental distinction with the instant invention inasmuch as the VP2 protein obtained under our invention is able to form empty chimeric capsids. In consequence of the above their immunogenic and hemagglutinant capacity is clearly superior as the following description will show. This added capacity of the VP2 protein obtained with our invention is further convenient in that other viral protein epitopes can be introduced in the said capsids by genetic manipulation of the recombinant baculoviruses, or by chemical manipulation of the actual capsids.

The advantages of VP2 protein synthesis in a baculovirus vector over E.coli synthesis are remarkable, namely a greater fidelity as to the identity of the protein obtained, a greater solubility of the product obtained, fusion proteins need not be used, etc. These are clearly advancements of and improvement to processes for obtaining recombinant subunit vaccines as regards previous processes.

WO 88/02026 discloses empty viral capsid vaccines, particularly empty CPV capsids consisting of all the structural proteins, expressed in a system of mammalian cells, wherein it is included the complete region of the CPU genome that contains all the genes encoding all the structural proteins [VP1 and VP2], using an oncogenic virus [Bovine papillomavirus (BPV)].

J. Gen. Virol. (1990), 71, 2747-2753 discloses the nucleotide sequence of feline panleukopenia virus (FPLV) and its comparison with the nucleotide sequence of CPV, including the sequence encoding CPV VP2.

### SUMMARY OF THE INVENTION

The present invention puts forth a new process for producing a recombinant subunit vaccine to protect dogs from CPV and cats from FPLV. The new vaccine produced thus contains empty CPV VP2 chimeric capsids consisting of autoassembled CPV recombinant VP2 proteins expressed in permissive insect cells infected with a recombinant baculovirus having the gene coding for the CPV VP2 protein. Said protein is hereinafter sometimes referred to as VP2 hereof.

The VP2 protein hereof is singularly characterised in forming empty chimeric capsids, optionally incorporating other viral protein epitopes by genetic manipulation of the recombinant baculoviruses or chemical manipulation of the actual capsids.

The object of the invention is therefore a new process for obtaining new improved subunit vaccines capable of protecting dogs and cats respectively from CPV and FPLV infections. As aforesaid the said vaccines contain the empty chimeric capsids formed by the said VP2 protein hereof, inasmuch as the said empty capsids have an enhanced hemagglutinant activity and are highly immunogenic, excelling other recombinant proteins of these viruses produced heretofore in any other vector. The new vaccines the invention provides and being one of its objects contain the said empty capsids with an immunologically acceptable diluent, with or without an adjuvant.

Since the said chimeric capsids can be chemically or genetically manipulated to introduce other unrelated viral protein or peptide epitopes therein, the use of the said capsids both for CPV and FPLV vaccinal purposes and modified to incorporate other epitopes, thereby to provide a polyvalent vaccine, are further additional objects of this invention.

The chimeric capsids provided by the invention can be useful in diagnosis to detect the presence of specific CPV antibodies or to induce polyclonal or monoclonal antibodies capable of CPV detection. The use of the VP2 protein hereof and of the chimeric capsids for the above purposes is a further object of the present invention.

An additional object of this invention is a recombinant baculovirus and the process for obtaining the same, capable of producing a CPV VP2 recombinant protein identical to the viral protein, as shown in antigenic reactivity assays and other biological functionality assays. The recombinant baculovirus was called AcMNPV.pCPVEx17 and filed on 2.3.91 with the European Collection of Animal Cell Cultures, (ECACC), at Porton Down, Salisbury, Wiltshire SP4 OJG (Great Britain), accession number V91030212.

A further object of the invention is the new baculovirus transfer vector (pCPVEx17) containing the nucleic acid sequence coding for the VP2 hereof. With a process known as AcMNPV wild strain homologous recombination this new vector leads to the said AcMNPV.pCPVEx17 recombinant baculovirus.

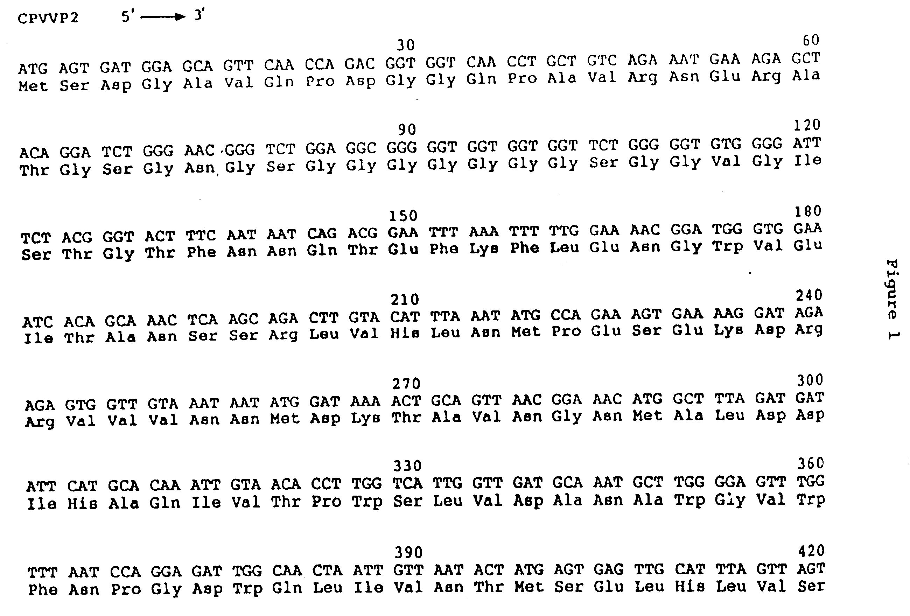

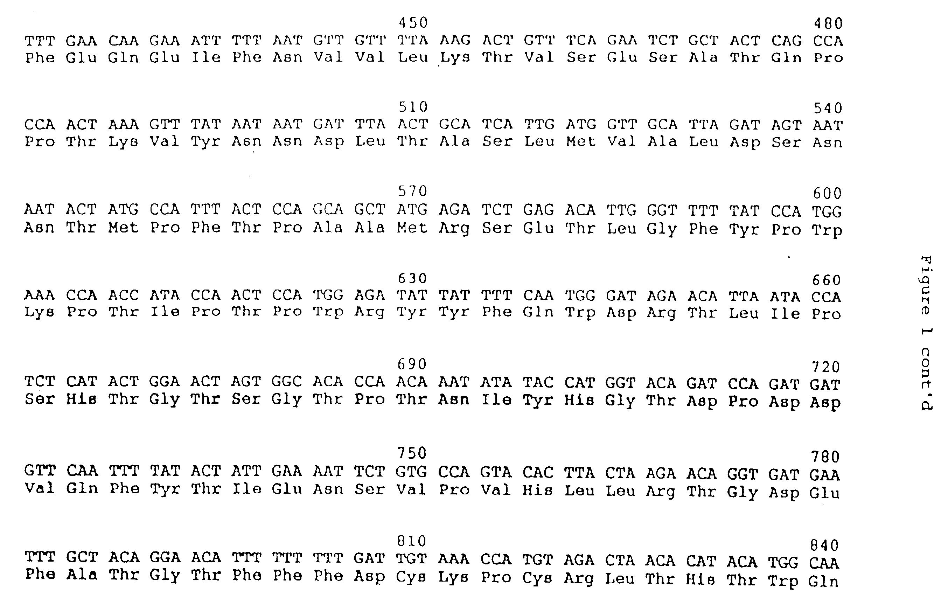

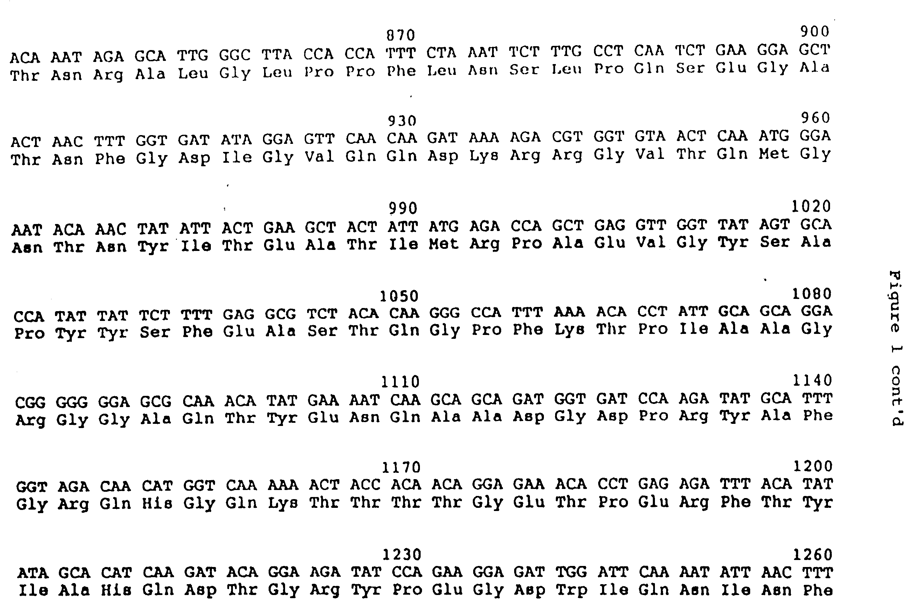

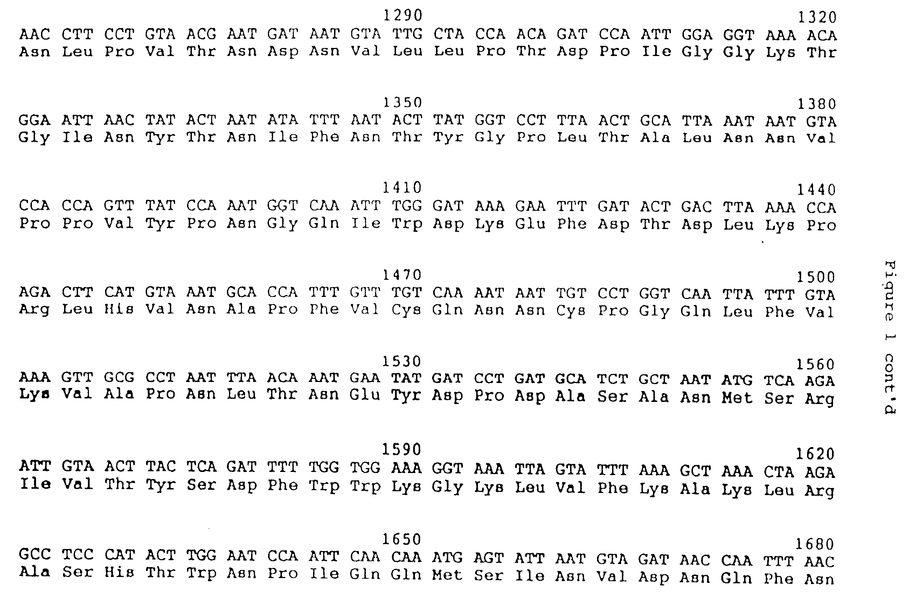

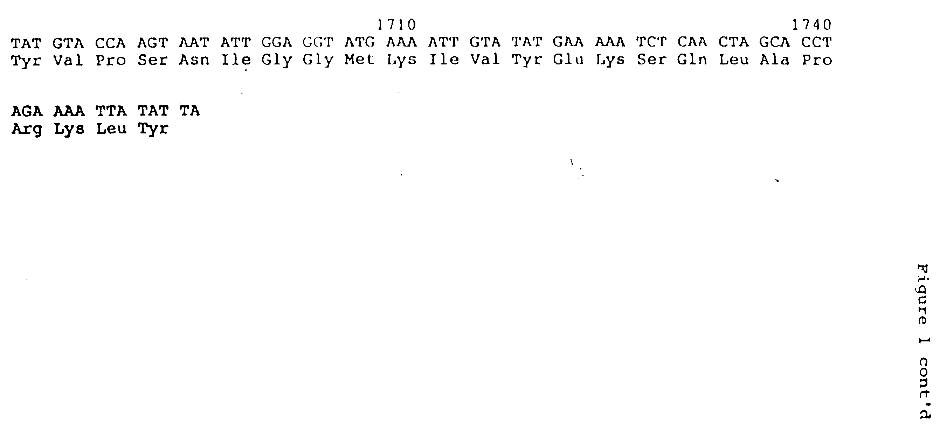

This invention also discloses the nucleic acid sequence coding for the VP2 protein of the invention (figure 1).

The empty CPV chimeric capsids formed by autoassembly of the CPV recombinant VP2 proteins expressed in permissive insect cells infected with a recombinant baculovirus having the DNA sequence coding for the CPV VP2 protein are yet another object of this invention.

### BRIEF DESCRIPTION OF THE FIGURES

Figure 1 shows the nucleotide sequence coding for the VP2 hereof and the amino acid sequence thereof. The nucleotide sequence is shown in direction 5' _{>>>>>}6 3' from left to right. The amino acids have been designated using the generally accepted three-letter code.

Figure 2 shows the construction of the pCPVEx17 transfer vector, pointing out the appropriate manipulations for inserting the CPV VP2 gene in the pJVP10Z plasmid.

Figure 3 shows the presence of empty chimeric capsids formed by aggregation of the VP2 protein hereof, as observed under an electron microscope.

### DETAILED DESCRIPTION OF THE INVENTION

The invention provides a new process for obtaining a recombinant subunit vaccine serving to protect against infections due to Canine Parvovirus or like viruses such as FPLV. The new vaccine contains the empty CPV chimeric capsids consisting of autoassembled CPV recombinant VP2 proteins expressed in permissive insect cells infected with a recombinant baculovirus having the gene coding for the CPV VP2 protein.

The invention also provides a recombinant baculovirus capable of expressing the CPV VP2 when inoculated in a permissive host, and the process for obtaining the said recombinant baculovirus.

The obtention of the recombinant baculovirus basically comprises the following steps:
a) Preparing the gene coding for the CPV VP2;
b) inserting the VP2 gene in a baculovirus transfer vector;
c) transfecting permissive host cells with the said baculovirus transfer vector holding the VP2 gene; and
d) selecting the recombinant baculovirus expressing the CPV VP2 protein.

The recombinant baculovirus obtained thus and the proteins and capsids produced are additionally characterised.

These steps will be described in detail hereinafter.

In a preferred embodiment the gene coding for the CPV VP2 protein is prepared in accordance with the protocol set forth in above-mentioned Spanish patent number ES 2029938 and inserted in locus Nhe I of the AcMNPV derived pJVP10Z plasmid, thereby to obtain a baculovirus transfer vector. In our invention the pCPVEx17 vector proved to have the CPV DNA adequately oriented to be expressed by the AcMNPV virus polyhedrine promoter.

The pCPVEx17 vector was used to co-transfect permissive host cells, with the AcMNPV virus wild strain DNA. Reference could, inter alia, be made to cells of lepidotera or their larvae. In a preferred embodiment of this invention Spodoptera frugiperda (S. frugiperda) cells, generally from the Sf9 strain, were transfected with pCPVEx17, though it can naturally be assumed that similar results would be achieved transfecting other permissive cells for recombinant baculovirus replication.

After transfection, the recombinant baculoviruses were selected after removing and titrating the floats produced in confluent monolayers of S. frugiperda cells. The blue plates with no trace of the viral polyhedrine under a light microscope were collected and back-titrated on S. frugiperda cells to obtain the recombinant baculoviruses. The AcMNPV.pCPVEx17 recombinant baculovirus is capable of expressing the CPV VP2 recombinant protein (VP2 hereof) and was filed with the ECACC, accession number V91030212.

A Dot Blot assay was used to verify that the VP2 gene had been adequately integrated into the said recombinant baculovirus genome.

The proteins expressed by the S. frugiperda cells infected with the recombinant baculovirus were analysed by electrophoresis in SDS-polyacrylamide 8% to 15% gradient gels and were tinted with Coomasie blue to observe a major presence of a protein with a virtual molecular weight of 67 KDa, equivalent to that of the viral VP2 in the recombinant virus plate. Immunodetection assays showed that the anti-CPV polyclonal antisera reacted with the VP2 expressed by the recombinant baculovirus. It was also found that neutralising monoclonal antibodies also recognised the recombinant VP2. It can in light of these results be said that the VP2 hereof expressed by the recombinant baculovirus in S. frugiperda cells is antigenically undistinguishable from the viral VP2.

The VP2 protein obtained with the above-described process can be used for diagnosis purposes to detect the presence of specific CPV antibodies or to induce polyclonal or monoclonal antibodies capable of CPV detection. They can further be used to immunise animals to CPV and other related viruses. ELISA assays have shown that immunised animal sera recognised the viral antigens while hemagglutination inhibition assays (IHA) showed that sera from animals immunised with the purified VP2 protein obtained with this invention offered IHA titres equal to or greater than those obtained with other commercially available vaccines, though in some cases the response was faster and/or considerably greater when animals were immunised with the VP2 obtained with our process (see table I).

It has also been ascertained that the VP2 protein hereof expressed by the AcMNPC.pCPVEx17 recombinant baculovirus induces antisera capable of neutralising CPV and protecting cell monolayers up to a dilution of 1:2000 equivalent to sera from animals that are hyperimmunised or have recovered from natural infections.

Based on the results obtained the VP2 protein expressed by the recombinant baculovirus vector hereof can be used to be formulated in vaccines in order to protect animals from infections caused by CPV and/or related viruses. These vaccines can be both passive and active. A passive vaccine could be obtained immunising animals with the recombinant and purified VP2 hereof and then isolating polyclonal antibodies from the said VP2 which could when purified be used in therapeutic or prophylactic applications. An active vaccine can be prepared resuspending the VP2 hereof in an immunologically acceptable diluent with an adjuvant.

It was submitted above that the VP2 protein obtained with the process of this invention is peculiar in that it can be aggregated, working pursuant to our conditions, and form pseudo-viral empty chimeric capsids of regular and uniform structure and with a size of some 22 nm, as shown by electron microscopy. No-one had to date described the "in vitro" formation of pseudo-viral capsids in Canine Parvoviruses using only the VP2 protein thereof. This much allows the recombinant VP2 proteins obtained to be easily purified. Furthermore, the empty capsids formed by VP2 assembly have an enhanced hemagglutinant activity and are highly immunogenic, more so than other CPV recombinant proteins produced heretofore in other vectors. The said capsids can hence be formulated to be used in vaccines capable of protecting animals from infections caused by CPV and/or related viruses (FPLV). Broadly speaking, an active vaccine can be prepared resuspending the said capsids in an immunologically acceptable diluent, with or without an adjuvant. An important feature of these empty chimeric capsids, that could be obvious to someone skilled in the art, is that they can be chemically or genetically manipulated to introduce the protein epitopes of other viruses, infection of which is to be protected, thereby to work as a polyvalent vaccine.

Phosphate buffer saline (PBS) solutions or other like saline solutions could be used as an immunologically acceptable diluent. The adjuvant used could be alumina gel suspensions or other adjuvants regularly used in formulating vaccines.

### DETAILED DESCRIPTION OF A PREFERRED EMBODIMENT OF THE INVENTION. (EXAMPLE)

### 1. OBTAINING RECOMBINANT BACULOVIRUSES EXPRESSING THE CPV VP2 GENE

### 1.1. Preparing the VP2 gene

Isolation and clonation of the gen coding for the VP2 from the canine parvovirus genome was described in detail in aforesaid Spanish patent number ES 2029938. The said patent application describes the construction of a genomic clone containing a copy of the CPV genome from the 780916 CPV attenuated strain (Cornell University) by means of a process comprising clonation of the end 3', the central region and the end 5' into three plasmids that were then digested with the appropriate restriction enzymes to isolate the CPV genome fragments, that were isolated and bonded to each other. The structural protein pUC18 gene was cloned to obtain the pCPV12 plasmid to facilitate structural gene expression.

### 1.2. VP2 gene insertion in a baculovirus transfer vector

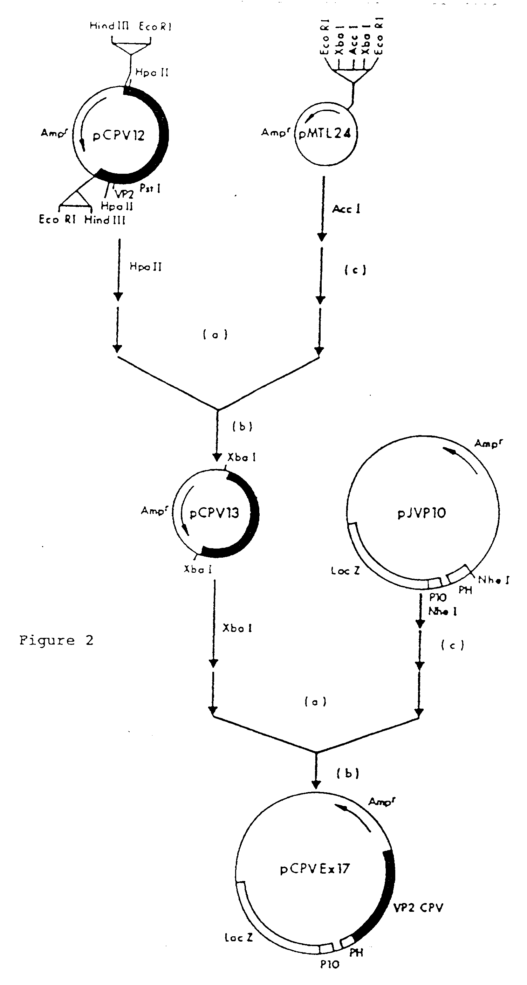

The plasmidic vector with AcMNPV derived NheI locus (pJVP10Z plasmid, Vialard, J. et al., J. Virol. 64, 37-50, 1990) was donated by Dr Chris Richardson (NRC. Quebec. Canada) and used to clone the Xbal fragment obtained from the pCPV12 as described in figure 2. The said figure shows how the pCPV12 HpaII fragment containing the gene coding for the CPV VP2 was cloned in pMTL24 vector AccI locus flanked by two Xbal loci, leading to the pCPV13 plasmid. The XbaI fragment of such plasmid was then inserted in pJPV10Z NheI locus. The plasmids obtained with the VP2 gene inserted were purified in accordance with the alkalysis method (Brimboim & Doly. Nucleic Acids Res. 7, 1513-1523. 1979) and characterised by restriction endonuclease mapping. The pCPVEx17 recombinant proved to have adequately oriented CPV DNA for expression thereof by the AcMNPV virus polyhedrine promoter.

### 1.3. Recombinant virus transfection and selection

S. frugiperda cells were transfected with infectious DNA mixtures purified with AcMNPV and pCPVEx17 plasmidic DNA in accordance with the process described by Burand et al. Virology 101. 286-290 (1980). AcMNPV DNA (1 µg) purified by the method of Smith and Summers (Virology 123, 393-406. 1983) was mixed with two different quantities of plasmidic DNA (1 and 5 µg) and taken to 750 µg with Hepes buffered saline solution (25 mM Hepes, pH 7.1, 140 mM NaCl and 125 mM CaCl₂). (The DNA solution was inoculated on monolayers of 2 x 10⁶ S. frugiperda cells and incubated for 4 h at room temperature. The floats were then removed and 5 ml of medium containing 10% calf foetal serum added. After incubating for 4 days the floats were collected and titrated in confluent monolayers of S. frugiperda cells. To improve detection of the recombinant plates the X-gal blue indicator was added to the agarose. Blue plates showing no traces of occlusive bodies (viral polyhedrine) under a light microscope were collected and back-titrated on S. frugiperda cells to obtain the recombinant viruses. After a third plating, high titre stocks of the recombinant viruses (10⁷⁻⁸ pfu/ml) were obtained.

The recombinant baculovirus was called AcMNPV.pCPVEx17 and filed with the European Collection of Animal Cell Cultures (ECACC), accession number V91030212.

### 2. DOT BLOT ASSAY

A Dot Blot assay was made to determine whether the VP2 gene had been integrated in the recombinant baculovirus genome, as follows.

To obtain DNA from the recombinant baculovirus the S. frugiperda cells were infected with the said virus at an infection multiplicity of 5 PFU/cell and incubated at 27°C for 48 hours. The infected cells were collected, sonicated and centrifuged at 1000 rpm for 10 min to eliminate cell remains. The floats were used as starting material for the assays.

A volume of 100 µl was denaturalised with 10 µl of NaOG 1 M, boiled for 5 min and placed immediately on ice. The mixture was neutralised with 10 µl of PO₄H₂Na 1M. A 20xSSC solution was added immediately to obtain a final 6xSSC concentration (SSC, citrate saline solution).

The solution was transferred to a nitrocellulose filter that had previously been moistened with 6xSSC. It was washed with more 6xSSC and dried at 37°C for 30 min. The DNA was fixed to the nitrocellulose filter under an U.V. light for 2-3 min. The membranes were then hybridised with a specific probe of the VP2 region marked with Phosphorous-32 at 37°C overnight. It was then washed with decreasing SSC solutions and autoradiographed.

A strong sign of hybridisation was observed only in the case of the cups containing floats from the culturesinfected with recombinant viruses, which indicated that the VP2 gene had been integrated in the viral genome.

### 3. PROTEIN AND IMMUNODETECION ANALYSIS

S. frugiperda cells were infected with recombinant baculovirus at a multiplicity of 5 PFU/cell and incubated at 27°C for 48 h. The cells were collected by centrifuging at 1000 rpm for 10 min, washed twice with phosphate buffered saline (PBS) solution pH 7.4 and resuspended at 1x10⁶ cells/ml with lysis buffer (5% sodium dodecyl sulphate (SDS), 1% β-mercaptoethanol and 17.4% glycerol). The samples were charged in SDS-polyacrylamide 8 to 15% gradient gels for electrophoresis and tinted with Coomasie blue or transferred to nitrocellulose membranes for immunodetection. The Coomasie blue tint showed the major presence of a protein with a virtual molecular weight of 67 KDa, equivalent to that of the VP2 viral protein in the recombinant virus plate.

For immunodetection the proteins were transferred to nitrocellulose membranes in accordance with previously described methods (Burnette, Anal. Biochem. 112. 195-203, 1981. Towbin et al., Proc. Natl. Acad. Sci. USA 76. 4350-4354. 1979). Protein transfer was made in a PhastSystem (Pharmacy) apparatus. In general 25 mA/gel were used for 10-15 minutes. The nitrocellulose strips were blocked with 3% powdered skim milk in Tris HCl 20 mM pH 7.5, NaCl 500 mM (TBS) for 30 min at room temperature. The strips were then incubated for an hour at room temperature with the first anti-CPV antiserum (specific antisera or monoclonal antibodies), washed with TBS-0.05% TWEEN-20™ (SIGMA CHEMICAL CO.) for 30 min at room temperature and incubated with anti-mouse rabbit serum where anti-CPV murine monoclonal antibodies was used as the first antibody at a dilution of 1:1000 for an hour at room temperature, or with anti-rabbit goat immunoglobulins marked with biotin (1:1000) where rabbit antisera was used as a first antibody. The strips were washed again and allowed to react with either protein A, marked with peroxidase, at a dilution of 1:1000 for an hour at room temperature, or streptavidin-peroxidase (1:2000) for 30 min at room temperature. After a thorough wash, the filters were developed with a TBS solution containing 0.5 mg/ml of 4-chloro-1-naphthol (Sigma), 17% (v/v) of methanol and 0.015% of hydrogen peroxide in TBS until visible bands appeared. The reaction was stopped treating the strips with distilled water.

Wherever specificities could be determined by immunoblot analysis all anti-CPV polyclonal antisera reacted with the VP2 protein expressed in baculovirus. The most significant feature is that all the neutralising monoclonal antibodies working in this assay also recognise recombinant VP2. This assay proves that the recombinant VP2 protein is antigenically undistinguishable from the viral VP2.

### 3.1. Recombinant protein and capsid purification

S. frugiperda cells were infected with AcMNPV.pCPVEx17 recombinant virus at an infection multiplicity of 5-10 PFU/cell and incubated at 27°C for 48-72 h. The cells were collected by centrifuging at 1000 rpm for 10 min, washed twice in a phosphate buffered saline solution pH 7.4 and resuspended at 2 x 10⁷ cells/ml in a 25 mM pH 9.5 bicarbonate buffer. The resuspended cells were broken down by sonication and centrifuged at 10,000 rpm for 10 min to eliminate cell remains. The floats containing the recombinant VP2 protein can be purified using its autoaggregating capacity to form empty capsids. They are to this end either purified by 20% ammonium sulphate precipitation or the empty capsids are centrifuged on CsCl gradients at 45,000 rpm for 14 h. Capsids offer a floatation density (P) of 1.30 g/cm³ when banded in CsCl gradient. Preparation purity was determined by electrophoresis in SDS-polyacrylamide gels as per the method described above and VP2 protein purity turned out to be in excess of 99%.

### 4. HEMAGGLUTINANT ACTIVITY

Recombinant protein functionality was analysed by a hemagglutination assay (HA). This activity is only associated to the particulate character of the product, thereby clearly distinguishing the same from previous ones. To this end 50 µl are mixed in a solution containing several factor two dilutions of a viral preparation with 50 µl of a solution containing 1% swine erythrocytes in pH 6.5 phosphate buffer and left at 4°C for at least 4 h in round bottom plates.

The results show that the preparation of VP2 capsids has an HA titre of 10⁵ units/ml.

### 5. CONFIRMING THE PRESENCE OF CAPSIDS BY ELECTRON MICROSCOPY

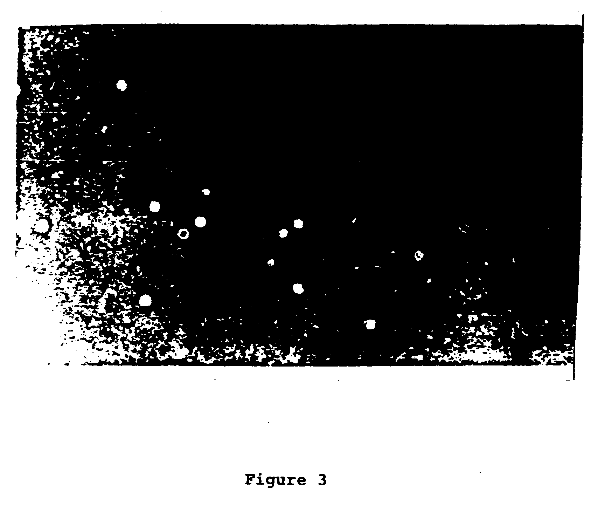

A purified VP2 preparation was tinted by negative contrast with 2% uranyl acetate and observed under an electron microscope at a magnifying power of 40,000 x 2.5, the presence of a large number of pseudo-viral chimeric particles of regular and uniform structure andwith a size of roughly 22 nm being observed. (Figure 3).

### 6. IMMUNIZING ANIMALS

Two New Zealand rabbits weighing 2 kg were intramuscularly immunised three times (days 0, 15 and 30) with 100 µg of a purified VP2 preparation. The first time in a complete Freund adjuvant, the second and third times with an incomplete adjuvant. A week after the last immunisation the rabbit was bled and the serum obtained tested with an ELISA assay, an hemagglutination inhibition assay (IHA) and an in vitro CPV neutralization assay, as described hereinbelow.
On the other hand, 22 Beagle dogs, aged 45 days, grouped into four batches, were subcutaneously immunized with two doses of semi-purified VP2 capsid preparations (with β-galactosidase). Protein concentration was calculated by the Bradford assay. The record dose was provided 28 days after the first immunization. The antigen was adjuvanted with ALHYDROGEL™ (SUPERFOS, DENMARK), Quil A (SUPERFOS) (50 µg/dog) or a combination of both. The dogs were bled twice a week until two months after the last injection. Two dogs were immunized with an inactivated commercial vaccine. A sentinel dog was used as a negative control to check for accidental CPV exposure. The results are set out in table I. Dogs in group I were given 100 µg of VP2 capsids per inoculation. Groups II, III and IV were respectively given 50, 25 and 10 µg per immunization.

The presence of antibodies in the dog sera was determined by an ELISA assay and by their capacity to inhibit viral HA, according to a process described hereinafter. All dogs immunized with our antigen displayed anti-CPV antibodies at various levels at the different adjuvant combinations considered (Table I). The highest antibody titres were nevertheless obtained when QuilA was used, alone or combined with ALHYDROGEL™. This combination was most effective at low doses (10 µg) of VP2 capsids.
HI and neutralization assays were used to assess the capacity of these preparations to induce CPV protection in dogs. Pollock and Carmichael (Cornell Vet. 72, 16-35, 1982) had previously shown that there is a good correlation between HI titre and protection; dogs showing HI>80 titres are considered refractory to CPV infection. All dogs vaccinated with our preparation showed high HI titres, between 150 and 5,120 (Table I). Sufficient HI titres lasted for over 2 months. A good correlation was found between HI titres and monolayer protection neutralisation. The titres obtained are in both cases far higher, even at lower doses (10 µg/dog) than what is needed to obtain a protective response in dogs. The titres were similar to those achieved with sera of dogs who had recovered from parvovirosis infection and much higher than those obtained with the vaccinated control. No anti-CPV antibodies were detected in the serum of dogs that were not inoculated.

### A) ELISA

The presence of specific CPV antibodies in the serum of immunized animals was determined by means of an indirect ELISA assay. The antigen used was both purified virus and purified VP2 protein. Briefly, polystyrene plates were coated with 0.5 µg of virus or 0.25 µ/well of VP2 in 100 µl of carbonate buffer (0.05 M, pH 9.6) at 4°C overnight. The plates were washed with PBS (NaCl 0.15 M in sodium phosphate 0.1 M pH 7.4) containing 0.05% Tween-20 and incubated with the antiserum for 2 h at 37°C, washed again and incubated with either anti-rabbit goat IgC marked with biotin, where rabbit sera was used, or with A protein marked with peroxidase where dog sera was used, for 1 h at room temperature. The antibody marked with biotin was later incubated with streptavidin marked with peroxidase for 30 min at room temperature. The plates were washed again and the reaction developed with o-phenylenediamine as a substrate for the peroxidase, for 10 min in darkness, and read at 450 nm in a multichannel spectrophotometer. The results obtained were as follows:

For the rabbit sera, the ELISA titre obtained was ¹/6400 for the complete virus.

For the dog sera, the antisera recognized the viral antigen in an ELISA assay perfectly, with complete virus titres of up to ¹/5120 for the 100 µg dose.

### B) HEMAGGLUTINATION INHIBITION (IHA)

The sera were inactivated at 56°C for 30 minutes and adsorbed with 0.25% Kaolin for 1 h at room temperature whereupon the immunised dog sera was diluted (factor two) in a phosphate buffer pH 6.5, starting with a 1:20 dilution. 25 µl of each of the previous dilutions were mixed with 25 µl of a virus dilution containing 4 units of HA, and incubated for 2 h at 37°C. 50 µl of 1% swine erythrocytes were then added in a phosphate buffer pH 6.5 and left at 4°C for at least 4 h in round bottom plates.

### C) "In vitro" CPV NEUTRALISATION

Neutralisation was determined by means of a monolayer protection test. Briefly, a known quantity of CPV (100 units of HA) was incubated with rabbit antiserum at different dilutions for 2 hours at 37°C. The samples were then inoculated on CRFK susceptible cell monolayers for 90 min at 37°C. The monolayers were covered with 1 ml of agarose at 1% in a fresh medium. Five days after infection the cells were fixed with 10% formaldehyde in PBS for 20 minutes, the agarose was removed and the remaining cells tinted with 1% violet crystal in 50% ethanol for 20 minutes. The level of protection was assessed by screening the infected monolayers.

The rabbit antiserum induced by the pCPVEx17 plasmid protein is able to protect the cellular monolayer from the infection up to a dilution of 1:2000, equivalent to sera from animals that are hyperimmunised or have recovered from natural infections.

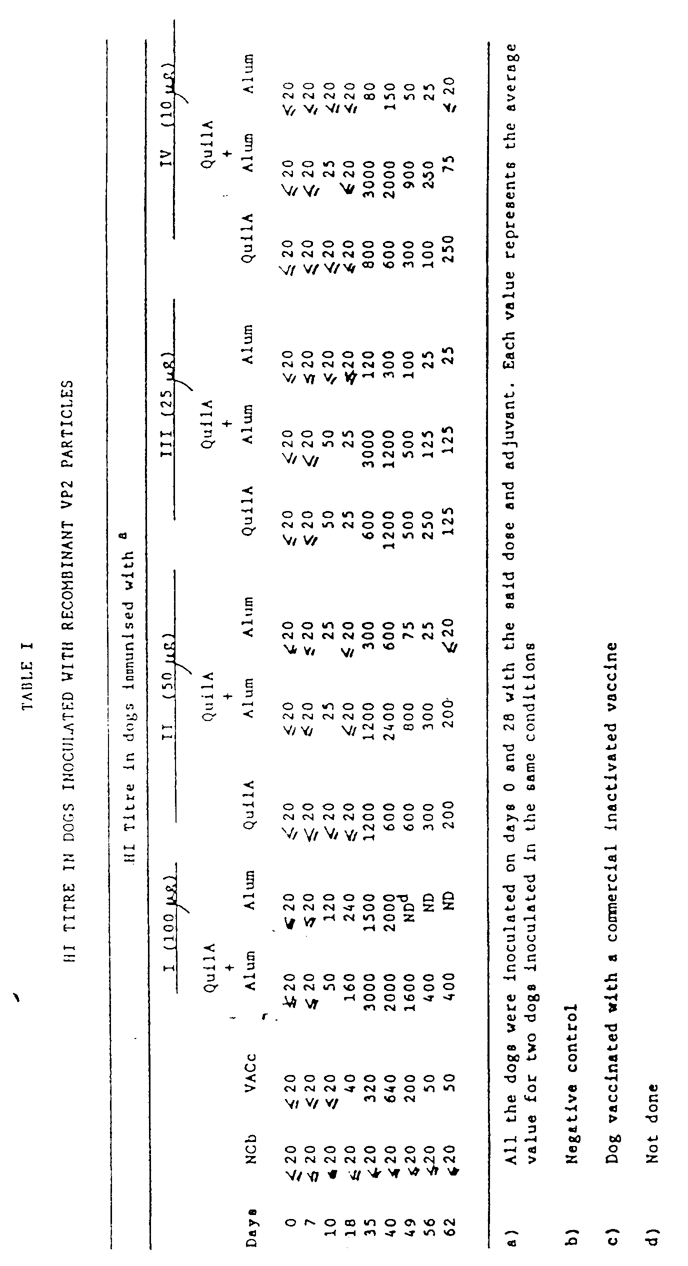

### 7. PROTECTING DOGS IMMUNISED WITH VP2 CAPSIDS FROM VIRULENT CPV VIRUS INFECTION

In order to determine the capacity of the VP2 recombinant particles to induce protection in dogs, six dogs were infected 6 weeks after the record injection with 1 ml of virulent CPV infected dog faeces, diluted twice in PBS buffer by oral-nasal inoculation. The clinical signs of the disease were monitored for 17 days post infection. Rectal temperatures were recorded from day 3 post infection. Blood samples were taken at intervals and checked for the presence of viral antibodies and for HA viremia and infection of susceptible cell cultures (Table II). The virus recovered from the dog faeces was identified as CPV by hemagglutination. All dogs immunised with the viral antigen were immune to viral infection. None of them developed any evincible clinical symptom of disease or viremia. The sentry and the vaccinated dogs however offered an enhanced humoral response which indicated viral replication.

Given the significant genetic and immunologic relationship between CPV and the feline panleukopenia virus (cats) it could reasonably be said that the same VP2 particles may be used to render cats immune to parvovirus, as is the case with conventional vaccines.

**TABLE II**

| IHA TITRE FOR DOGS STIMULATED WITH VIRULENT CPV | | | | |
|---|---|---|---|---|
| DOGS | Post-challenge days | | | |
| | 0 | 7 | 10 | 17 |
| 1 | - | 400 | 3200 | 1600 |
| 2 | - | 1600 | 3200 | 1600 |
| 3 | 400 | 400 | 400 | 400 |
| 4 | 400 | 400 | 400 | 400 |
| 5 | 400 | 200 | 200 | 200 |
| 6 | 400 | 200 | 200 | 200 |

The dogs used in the challenge test were immunised as follows: 1. Sentry dog. 2. Dog vaccinated with an inactivated commercial vaccine. 3. Dog vaccinated with 50 µg of VP2 adjuvanted with QuilA. 4. Dog vaccinated with 50 µg of VP2 adjuvanted with alumina and QuilA. 5. Dog vaccinated with 25 µg of VP2 adjuvanted with alumina and QuilA. 6. Dog vaccinated with 10 µg of VP2 adjuvanted with alumina and QuilA.

### 8. VACCINE FORMULATION

A passive vaccine can be obtained immunising animals with the purified recombinant VP2 vaccines as described herein. Polyclonal antibodies directed against this VP2 can be isolated from serum, milk or other animal bodily fluids. These antibodies can then be purified and used for therapeutic or prophylactic applications.

An active vaccine can be prepared resuspending the recombinant VP2 capsids described herein in an immunologically acceptable diluent such as PBS and an adjuvant such as Alhydrogel or QuilA. Initial and record injections or oral administration of the vaccinal solution can be used to confer immunity.

An active vaccine can also be prepared resuspending the empty capsids in an immunologically acceptable diluent with or without an adjuvant. Anyone skilled in the art will clearly see that these chimeric capsids formed only by VP2 can be chemically or genetically manipulated to introduce other viral protein epitopes and hence function as a polyvalent vaccine.

### 9. CONCLUSIONS

The AcMNPV.pCPVEx17 baculovirus is capable of producing a recombinant VP2 absolutely identical to the viral VP2 protein, as shown with the DNA sequence, molecular weight estimate and antigenic characterisation. The VP2 obtained herein with our process is also remarkably capable of forming empty capsids, thereby providing the same with a hemagglutinant and immunogenic activity that is clearly greater than in other previously described recombinant proteins, as shown with the animal immunisation tests herein described.

This enhanced immunogenic capacity can be used by those skilled in the art to present other viral protein epitopes, that can be introduced therein by either chemical or genetic manipulation of the recombinant baculoviruses.

### Translation of the keys to the figures

Figure 2
(a) Isolate agarose gel fragments.
(b) Bond.
(c) Phosphatase.

Having described the object of the present invention what is deemed as the essence thereof is now contained in the following

## Claims

1. A recombinant subunit vaccine to protect dogs against canine parvovirus (CPV) infection, comprising:
a) an immunising quantity of empty CPV VP2 chimeric capsids formed by autoassembly of CPV recombinant VP2 proteins; and
b) a diluent and, optionally, an adjuvant, being immunologically acceptable.

2. The vaccine as in claim 1, wherein said empty chimeric capsids are obtainable by the autoassembly of the CPV recombinant VP2 protein produced during replication in permissive insect cells of a recombinant baculovirus with the gene coding for the CPV recombinant VP2 protein integrated in its genome.

3. The vaccine as in claim 2, wherein said recombinant baculovirus is identified as AcMNPV.pCVEx17 and filed with ECACC, accession number V91030212.

4. A recombinant subunit vaccine to protect cats against feline panleukopenia virus (FPLV) infection, comprising:
a) an immunising quantity of empty CPV VP2 chimeric capsids formed by autoassembly of CPV recombinant VP2 protein; and
b) a diluent and, optionally, an adjuvant, being immunologically acceptable.

5. The vaccine as in claim 4, wherein said empty chimeric capsids are obtainable by the autoassembly of the CPV recombinant VP2 protein produced during replication in permissive insect cells of a recombinant baculovirus with the gene coding for the CPV recombinant VP2 protein integrated in its genome.

6. The vaccine as in claim 5, wherein said recombinant baculovirus is identified as AcMNPV.pCVEx17 and filed with ECACC, accession number V91030212.

7. Empty CPV VP2 chimeric capsids consisting of autoassembled CPV recombinant VP2 proteins expressed in permissive insect cells infected with a recombinant baculovirus having the gene coding for the CPV VP2 protein.

8. Empty capsids as in claim 7, having a hemagglutination capacity and being immunogenic.

9. Empty capsids as in claim 7, wherein said recombinant baculovirus is identified as AcMNPV.pCVEx17 and filed with ECACC, accession number V91030212.

10. Use of the empty CPV VP2 chimeric capsids according to any one of claims 7 to 9 for *in vitro* detecting the presence of CPV specific antibodies.

11. A recombinant baculovirus which expresses CPV recombinant VP2 protein in permissive insect cells, the recombinant protein expressed being capable of being autoassembled to form empty CPV chimeric capsids consisting of CPV recombinant VP2, said recombinant baculovirus being identified as AcMNPV.pCVEx17 and filed with ECACC, accession number V91030212.

## Patentansprüche

1. Ein rekombinanter Mehzweckimpfstoff, dazu geeignet, Hunde vor einer Infektion durch Parvovirus Canino (CPV) zu schützen, der einschließt:
a) eine immunisierende Menge von leeren chimärischen VP2-Kapsiden des CPV, die durch Selbstzusammenfügung von rekombinanten VP2-Proteinen des CVP gebildet werden; und
b) ein Lösungsmittel und wahlweise ein Hilfsstoff, beide immunologisch akzeptabel.

2. Impstoff gemäß Anspruch 1, in dem die genannten chimärischen Kaspside durch Selbstzusammenfügung des rekombinanten VP2-Proteins wahrend der Vermehrung in permissiven Insektenzellen eines rekombinanten Baculovirus, das dasjenige Gen, das das rekombinante VP2-Protein des CPV kodiert, in seiner Gensequenz besitzt, gewonnen werden können.

3. Impfstoff gemäß Anspruch 2, in dem das genannte rekombinate Baculovirus dasjenige ist, das als AcMNPV.pCVEx17 bei der ECACC unter der Depotnununer V91030212 hinterlegt ist.

4. Ein rekombinanter Mehrzweckimpfstoff, dazu geeignet, Katzen vor einer Infektion durch den Virus Panleucopenia Felina (FPLV) zu schützen, der einschließt:
a) eine immunisierende Menge von leeren chimärischen VP2-Kapsiden des CPV, die durch Selbstzusammenfügung von rekombinanten VP2-Proteinen des CVP gebildet werden; und
b) ein Lösungsmittel und wahlweise ein Hillsstoff, beide immunologisch akzeptabel.

5. Impfstoff gemäß Anspruch 4, in dem die genannten chimärischen Kaspside durch Selbstzusammenfügung des rekombinanten VP2-Proteins während der Vermehrung in permissiven Insektenzellen eines rekombinanten Baculovirus, das dasjenige Gen, das das rekombinante VP2-Protein des CPV kodiert, in seiner Gensequenz besitzt, gewonnen werden können.

6. Impfstoff gemäß Anspruch 5, in dem dem das genannte rekombinate Baculovirus dasjenige ist, das als AcMNPV.pCVEx17 bei der ECACC unter der Depotnummer V91030212 hinterlegt ist.

7. Leere chimärische VP2-Kapside des CPV, die aus rekombinanten VP2-Proteinen bestehen, exprimiert in permissiven Insektenzellen, die mit einem rekombinanten Baculovirus infiziert sind, das das Gen besitzt, das das VP2-Protein des CPV kodiert.

8. Leere Kapsiden gemäß Anspruch 7, die die Fähigkeit zur Hämagglutination besitzen und immunogen sind.

9. Kapside gemäß Anspruch 7, in denen das genannte rekombinante Baculovirus dasjenige ist, das als AcMNPV.pCVEx17 bei der ECACC unter der Depotnummer V91030212 hinterlegt ist.

10. Einsatz der leeren chimärischen VP2-Kapside des CPV gemäß eines beliebigen der Ansprüche 7 bis 9, um in vitro die Anwesenheit von spezifischen Antikörpern gegen CPV nachzuweisen.

11. Ein rekombinantes Baculovirus, das das rekombinante VP2-Protein des CPV in permissiven Insektenzellen exprimiert, wobei das exprimierte rekombinante Protein fähig ist, sich selbst zusammenzufügen, um leere chimärische Kapside des CPV zu formen, die aus dem rekombinanten VP2-Protein des CPV bestehen, wobei das genannte rekombinante Baculovirus als AcMNPV.pCVEx17 identifiziert und unter der Depotnummer V91030212 bei der ECACC hinterlegt ist.

## Revendications

1. Un vaccin sous-unité recombinant adéquat pour protéger les chiens de l'infection causée par parvovirus (CPV), qui comprend:
a) une quantité immunisante de capsides chimériques vides de VP2 de CPV formées par autoassemblage de protéines VP2 recombinantes de CPV; et
b) un diluant et, optativement, un adjuvant, immunologiquement acceptables.

2. Vaccin selon la revendication 1, dans laquelle lesdites capsides chimériques vides peuvente être obtenues par autoassemblage de la protéine VP2 recombinante de CPV produite lors de la réplication en cellules d'insecte permissives d'un baculovirus recombinant ayant le gène codant pour la protéine VP2 recombinante de CPV2 intégré dans son génome.

3. Vaccin selon la revendication 2, dans laquelle ledit baculovirus recombinant est celui identifié par AcMNPV.pCVEx17 es déposé à la ECACC sous le numéro de dépôt V91030212.

4. Un vaccin sous-unité recombinant adéquat pour protéger les chats de l'infection causée par le virus de la panleucopénie féline (FPLV), qui comprend:
a) une quantité immunisante de capsides chimériques vides de VP2 de CPV formées par autoassemblage de protéines VP2 recombinantes de CPV; et
b) un diluant et, optionnellement, un adjuvant, immunologiquement acceptables.

5. Vaccin selon la revendication 4, dans laquelle lesdites capsides chimériques vides peuvent être obtenues par autoassemblage de la protéine VP2 recombinante de CPV produite lors de la réplication en cellules d'insecte permissives d'un baculovirus recombinant ayant le gène codant pour la protéine VP2 recombinante de CPV intégré dans son génome.

6. Vaccin selon la revendication 5, dans laquelle ledit baculovirus recombinant est celui identifié par AcMNPV.pCVEx17 et déposé à la ECACC sous le numéro de dépôt V91030212.

7. Capsides chimériques vides de VP2 de CPV consituées par des protéines VP2 recombinantes de CPV exprimées en cellules d'insecte permissives infectées avec un baculovirus recombinant ayant le gène codant pour la protéine VP2 de CPV.

8. Capsides vides selon la revendication 7, qui a la capacité d'hémagglutination et elles sont immunogéniques.

9. Capsides selon la revendication 7, dans lesquelles ledit baculovirus recombinant est celui identifié par AcMNPV.pCVEx17 et déposé à la ECACC sous le numéro de dépôt V9103212.

10. Emploi des capsides chimériques vides de VP2 de CPV selon n'importe laquelle des revendications 7 à 9 pour détecter in vitro la présence d'anticorps spécifiques face à CPV.

11. Un baculovirus recombinant qui exprime la protéine VP2 recombinante de CPV en cellules d'insecte permissives, la protéine recombinante exprimée étant capable d'être autoassemblée pour former des capsides chimériques vides de CPV constituées par la protéine VP2 recombinante de CPV, ledit baculovirus recombinant étant identifié par AcMNPV.pCVEx17 et déposé à la ECACC sous le numéro de dépôt V91030212.
